# EUROPÄISCHE PATENTSCHRIFT

(11) **EP 0 317 492 B1**
(45) Veröffentlichungstag und Bekanntmachung des Hinweises auf die Patenterteilung: **01.06.1994**
(21) Anmeldenummer: 88730249.5
(22) Anmeldetag: 11.11.1988
(51) Int. Cl.: C07C 233/67, A61K 49/04

(54) **Neue substituierte Dicarbonsäure-bis(3,5-dicarbamoyl-2,4,6-triiodanilide), Verfahren zu deren Herstellung sowie diese enthaltende Röntgenkontrastmittel**
Substituted dicarboxylic acids-bis(3,5-dicarbamoyl-2,4,6-triiodo-anilides), process for their preparation and X-ray contrast agents containing them
Acides dicarboxyliques-(bis 3,5-dicarbamoyl-2,4,6-triiodo-anilide) substitués, procédé de leur préparation ainsi que les agents de contraste aux rayons X les contenant

(30) Priorität: 16.11.1987 DE 3739098
(43) Veröffentlichungstag der Anmeldung: 24.05.1989
(73) Patentinhaber: SCHERING AKTIENGESELLSCHAFT, 13342 Berlin (DE)
(72) Erfinder: Blaszkiewicz, Peter, Dr., D-1000 Berlin 41 (DE); Speck, Ulrich, Prof. Dr., D-1000 Berlin 28 (DE)

(56) Entgegenhaltungen:
- EP-A- 0 023 992
- EP-A- 0 049 745
- EP-A- 0 108 638

## Beschreibung

Die Erfindung betrifft neue substituierte Dicarbonsäure-bis(3,5-dicarbamoyl-2,4,6-triiodanilide) der allgemeinen Formel I 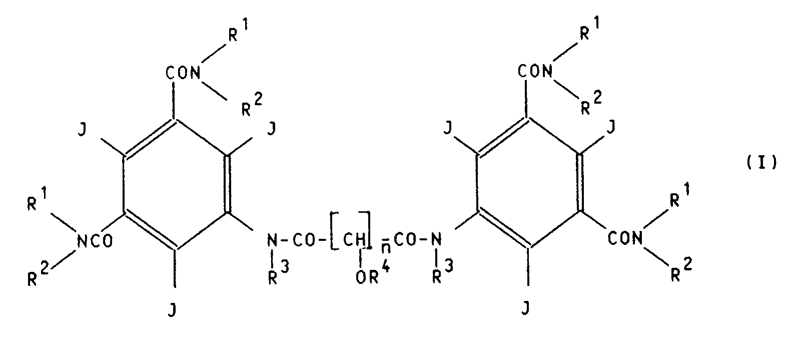
worin
R¹ ein Wasserstoffatom, einen C₁-C₄-Alkylrest oder R²,
R² einen gerad- oder verzweigtkettigen C₂-C₈-Mono- oder Polyhydroxyalkylrest,
R³ ein Wasserstoffatom, einen C₁-C₄-Alkylrest oder R²,
R⁴ ein Wasserstoffatom oder einen C₁-C₄-Alkylrest und
n = 1 oder 2
bedeuten,
Verfahren zu deren Herstellung
sowie diese enthaltende Röntgenkontrastmittel.

Die Reste R¹, R³ und R⁴ enthalten als niedere Alkylreste Ethyl-, Propyl- und Butylreste, besonders geeignet sind die Methylreste.

Der Rest R² enthält gerad- oder verzweigtkettige Mono- oder Polyhydroxyalkylreste mit 2 bis 8 Kohlenstoffatome, vorzugsweise 2 bis 5 Kohlenstoffatome. Geradkettige Reste von R² bestehen vorzugsweise aus 2 bis 4 Kohlenstoffatomen, verzweigtkettige vorzugsweise aus 3 bis 5 Kohlenstoffatomen. Die Hydroxygruppen im Rest R² können als primäre oder sekundäre Hydroxygruppen vorliegen. Der Rest R² kann 1 bis 5 Hydroxygruppen enthalten, bevorzugt sind 1 bis 3 Hydroxygruppen. Als Rest R² sei beispielsweise genannt:
Der 2-Hydroxyethyl-, 2-Hydroxypropyl-, 3-Hydroxypropyl-, 2,3-Dihydroxy-propyl, 1-Hydroxymethyl-2-hydroxy-ethyl-, 2,3-Dihydroxybutyl-, 2,4-Dihydroxybutyl-, 3,4-Dihydroxybutyl-, 3-Hydroxy-2-(hydroxymethyl)-propyl-, 2,3-Dihydroxy-1-methylpropyl-, 2-Hydroxy-3-(hydroxymethyl)-butyl-, 2,3,4-Trihydroxy-butyl-, 2,4-Dihydroxy-3-(hydroxymethyl)-butyl-, 3-Hydroxy-2,2-bis-(hydroxymethyl)-propyl-, 4-Hydroxy-3,3-bis-(hydroxymethyl)-butyl-, 4-Hydroxy-2,2-bis-(hydroxymethyl)-butyl-, 2-Hydroxy-1,1-bis-(hydroxymethyl)-ethyl-, 1,3-Dihydroxy-isopropyl-,2,3-Dihydroxy-1-hydroxy-methyl-propyl-Rest.

Röntgenkontrastmittel sind unentbehrliche Hilfsmittel bei der Diagnose zahlreicher Erkrankungen, wie z.B. von arteriosklerotischen Gefäßprozessen, Tumoren, Infarkten, Erkrankungen der Nieren und ableitenden Harnwege. Seit der Einführung der ersten Produkte sind große Fortschritte erzielt worden:
Die chemotoxischen Eigenschaften der Kontrastmittel wurden stark vermindert. Für die klinische Anwendung bedeutet das ein geringeres Auftreten von Nebenwirkungen wie Übelkeit, Erbrechen, von bestimmten Kreislaufreaktionen, Urticaria, Bronchospasmus und anderen Symptomen bis hin zum Schock und Tod. Pharmakologisch sind chemotoxische Effekte z.B. als LD₅₀ nach intravenöser Injektion meßbar.

Die früher benutzten Produkte waren sehr stark hyperton (z.B. bis zur 8-fachen Osmolalität des Blutes) und verursachten dementsprechend eine große Zahl von z.T. schwerwiegenden Nebenwirkungen wie z.B. Blutdruckabfall, Bradycardie bis hin zum Herzstillstand, Störungen der Blut-Hirn-Schranke, starke Schmerzen usw.. Neuere Kontrastmittel weisen in den klinisch gebräuchlichen Konzentrationen nur noch die 2- bis 3-fache Osmolalität des Blutes auf.

Obwohl also sowohl die Chemotoxizität als auch die Hypertonizität der Kontrastmittel gesenkt wurden, konnten bis heute keine idealen Werte erreicht werden.

Selbst die neuesten sogenannten nicht-ionischen Kontrastmittel verursachten noch schwere und schwerste Zwischenfälle (McClennan, Radiology 162, 1:1-8 [1987]: "Low-osmolality contrast media: Premises and Promises"), die auf chemisch-toxische Wirkungen zurückgeführt werden müssen.

Auch die Osmolalität dieser Produkte ist noch viel zu hoch, als daß man von physiologischen Kontrastmitteln sprechen könnte. Es ist daher nicht verwunderlich, daß zumindest ein bestimmter Prozentsatz der Patienten über starke Schmerzen bei der Untersuchung mit diesen Produkten klagt ("Pain and hemodynamic effects in aortofemora angiography" in Acta Radiol. Diagnosis 23,4:389-399 [1982]).

Erfahrungsgemäß lassen sich diese Probleme durch die Synthese von wasserlöslichen, sehr hydrophilen "nichtionischen Dimeren", d.h. von Kontrastmittelmolekülen, die aus der Verknüpfung von 2 triiodierten Aromaten bestehen, weitgehend lösen. Solche Substanzen wurden erstmals in der DOS 26 28 517 beschrieben. Seither sind eine Reihe von sehr ähnlichen Strukturen beschrieben worden, z.B. in DOS 28 05 928, EP 0023992, EP 0049745 und EP 0108638.

Nichtionische Dimere sind im allgemeinen bei allen für die Röntgendiagnostik gebräuchlichen Konzentrationen im Vergleich zu den Körperflüssigkeiten nicht hyperton. Darüber hinaus weisen einige Vertreter dieser Substanzklasse eine sehr geringe Chemotoxität auf, d.h. es werden extrem hohe LD₅₀-Werte nach intravenöser Injektion erzielt.

Trotz dieser Vorzüge haben Kontrastmittel auf Basis nichionischer Dimerer bisher kaum klinische Anwendung gefunden. Die Ursache dafür ist die Viskosität insbesondere der hochkonzentrierten Lösungen, die für bestimmte besonders kritische angiographische Untersuchungen benötigt werden. So sind selektiv angiographische Untersuchungen der Herzkranzgefäße und Ventrikel nur mit Kontrastmittellösungen, die 350 mg Iod/ml oder mehr enthalten, durchzuführen.

Dabei müssen die Kontrastmittellösungen mit sehr hoher Geschwindigkeit durch etwa 100 cm lange sehr enge Katheter injiziert werden. Lösungen mit über 12 bis 15 cP bei 37° C sind dafür kaum mehr geeignet. Aber auch für die sehr rasche intravenöse Injektion, wie sie für verschiedene moderne Röntgentechniken notwendig ist, sind sehr gut verträgliche und wenig visköse Kontrastmittel erforderlich.

Die Viskosität der nicht ionischen dimeren Kontrastmittel ist von einer Reihe von Faktoren abhängig, von denen der Iodgehalt der Moleküle eine wesentliche Rolle spielt. Bei steigendem Iodgehalt nimmt die Viskosität der Lösungen der betreffenden Moleküle ab, gleichzeitig aber auch ihre Löslichkeit in Wasser.

Aufgabe der vorliegenden Erfindung war es daher, sehr gut verträgliche und wasserlösliche, bei hohen Konzentrationen blutisotone und gleichzeitig wenig visköse Kontrastmittel mit hohem Iodgehalt zur Verfügung zu stellen.

Es war überraschend, daß eine vergleichsweise geringe chemische Veränderung gegenüber dem bekannten Stand der Technik zu den erfindungsgemäßen Verbindungen der Formel I führte, deren wässrige Lösungen neben ausgezeichneter Verträglichkeit und Blutisotonie auch bei Konzentration von 300 bis 400 mg Iod/ml die erwünschte ausreichend niedrige Viskosität besitzen, um eine universelle Anwendung in der Angiographie sowohl bei schneller Zugabe als auch bei der Applikation hoch konzentrierter Lösungen durch enge Katheter zu ermöglichen.

Die erfindungsgemäßen Verbindungen unterscheiden sich strukturell dadurch vom bekannten Stand der Technik, daß beide Benzolkerne durch Hydroxy- oder C₁-C₄-Alkoxymalonsäureamide oder durch Hydroxy- oder C₁-C₄-Alkoxybernsteinsäureamide verknüpft sind.

Die erfindungsgemäßen Verbindungen der allgemeinen Formel I sind somit als schattengebende Substanzen hervorragend zur Herstellung von bzw. zur Anwendung in Röntgenkontrastmitteln geeignet. Die neuen Verbindungen besitzen alle Eigenschaften, die von Röntgenkontrastmitteln gefordert werden. Viele sind, obwohl nicht-ionisch, sehr gut wasserlöslich. Die neuen Verbindungen stellen hervorragend verträgliche Röntgenkontrastmittel dar, die in der Angiographie, Urographie, Myelographie, Lymphographie und zur Darstellung verschiedener Körperhöhlen und zu anderen radiologischen Untersuchungen geeignet sind.

Aufgrund ihres schwachen und neutralen Geschmackes eignen sich einige der Verbindungen hervorragend für die orale Applikation und zum Einbringen in die Lunge.

Der den gebräuchlichen Kontrastmitteln anhaftende bittere und Übelkeit auslösende Geschmack ist als schwerwiegender Nachteil insbesondere in der Gastrographie und Bronchographie anzusehen.

Die Erfindung betrifft somit auch neue Röntgenkontrastmittel auf Basis von Verbindungen der allgemeinen Formel I. Die Herstellung der neuen Röntgenkontrastmittel auf Basis der erfindungsgemäßen Verbindungen der allgemeinen Formel I erfolgt in an sich bekannter Weise, z.B. dadurch, daß man die schattengebende Substanz mit den in der Galenik üblichen Zusätzen, z.B. Stabilisatoren wie Natriumedetat, Calcium-dinatriumedetat, physiologisch verträglichen Puffern, Natriumchlorid u.ä., in eine für die intravenöse Applikation geeignete Form bringt. Die Konzentration der neuen Röntgenkontrastmittel im wässrigen Medium richtet sich ganz nach der röntgendiagnostischen Methode. Die bevorzugten Konzentrationen und Dosierungen der neuen Verbindungen bewegen sich in den Bereichen von 50-500 mg J/ml für die Konzentration und 5-500 ml für die Dosierung. Besonders bevorzugt sind Konzentrationen zwischen 100-400 mg J/ml.

Die Erfindung betrifft ferner ein Verfahren zur Herstellung von Verbindungen der allgemeinen Formel I, das dadurch gekennzeichnet ist, daß man in an sich bekannter Weise ein
substituiertes Dicarbonsäurederivat der allgemeinen Formel II 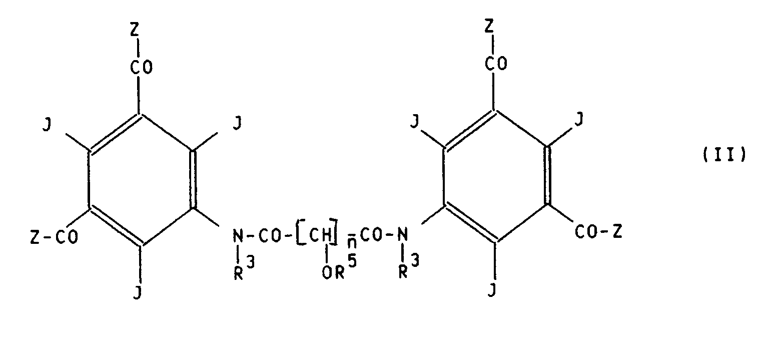
worin
R³ ein Wasserstoffatom oder eine C₁-C₄-Alkylgruppe,
R⁵ eine C₁-C₄-Alkylgruppe, Benzyl- oder eine C₁-C₆-Acylgruppe,
Z einen reaktiven Säure- oder Esterrest und
n= 1 oder 2
bedeuten,
mit einer Base der allgemeinen Formel III 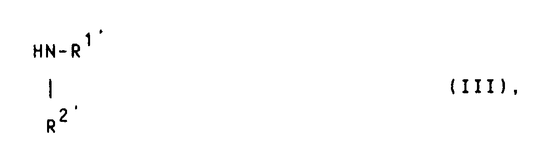
worin
R^{1'} ein Wasserstoffatom oder einen C₁-C₄-Alkylrest oder R^{2'} und
R^{2'} einen gerad- oder verzweigtkettigen C₂-C₈- Mono- oder Polyhydroxyalkylrest in freier oder geschützter Form
bedeuten,
umsetzt,
gegebenenfalls die aromatischen Acylaminogruppen zu C₁-C₄- N-Alkyl- oder N-Hydroxyalkyl-acylaminoverbindungen umsetzt und/oder gegebenenfalls die geschützten Hydroxylgruppen in Freiheit setzt.

Steht R⁵ in der allgemeinen Formel II für eine Acylgruppe so werden Monocarbonsäuren mit 1-6 Kohlenstoffatomen verwendet. Geeignet sind die Acylgruppen der Essig-, Propion-, Butter- und Benzoesäure, besonders geeignet ist die der Benzoesäure.

Als reaktive Säure- und Esterreste für Z finden die Säurehalogenide, besonders die Säurechloride und -bromide, aber auch andere, wie zum Beispiel die in Tetrahedron 36, 2409 (1980) publizierten Beispiele, Anwendung.

Für die Amidierungsreaktion der Verbindung der allgemeinen Formel II mit der Base der allgemeinen Formel III können die in den Gruppen R^{1'} und R^{2'} enthaltenen Hydroxylgruppen in freier oder geschützter Form vorliegen. Sollen diese Hydroxylgruppen in geschützter Form vorliegen, kommen alle Hydroxyschutzgruppen infrage, die bekanntermaßen für einen intermediären Hydroxylgruppenschutz geeignet sind, d.h. die sich leicht einführen und sich später unter Rückbildung der letztlich gewünschten freien Hydroxylgruppe auch wieder leicht abspalten lassen. Bevorzugt ist der Schutz durch Veresterung z.B. durch Einführung des Benzoyl- oder Acyl-, insbesondere des Acetylrestes. Geeignete Schutzgruppen sind auch Ethergruppen wie z.B. Benzyl-, Di- und Triphenyl-methyl-Ethergruppen sowie Acetal- und Ketalgruppen mit z.B. Acetaldehyd und Aceton.

Auch cyclische Ketale, wie zum Beispiel Dioxan- und Dioxepinderivate finden Anwendung. Besonders geeignet ist das 6-Amino-2,2-dimethyl-l,3-dioxepin-5-ol (Europäische Patentanmeldung, Veröffentlichungsnummer 0033426).

Die Amidierung der beiden Carboxylgruppen, die als reaktiver Säure- oder Esterrest Z vorliegen, erfolgt in einem geeigneten Lösungsmittel bei 0° bis 120° C, vorzugsweise bei 20° bis 100° C. Geeignete Lösungsmittel sind u.a. polare Lösungsmittel, wie beispielsweise Wasser, Dioxan, Tetrahydrofuran, Dimethylformamid, Dimethylacetamid, Hexametapol, Aceton u.ä. und deren Gemische. Da bei der Amidierungsreaktion pro umgesetzten Molekül der Verbindung der Formel II zwei Mol Säure (aus dem reaktiven Säure- oder Esterrest) frei werden, die neutralisiert werden müssen, benötigt man für jede reaktive Säure- oder Estergruppe zwei Äquivalente Base, zweckmäßigerweise im Überschuß von mindestens 10 %. Zur praktischen Durchführung wird die gelöste oder suspendierte Ausgangsverbindung der Formel II mit mindestens 4,4 Äquivalenten Base der Formel III oder mit mindestens 2,2 Äquivalenten Base der Formel III und zusätzlich mindestens 2,2 Äquivalenten einer von III verschiedenen Base versetzt, die dann als Protonenakzeptor dient. Als Protonenakzeptor verwendet man vorteilhaft tertiäre Amine, wie z.B. Triethylamin, Tributylamin, Pyridin oder Dimethylaminopyridin oder anorganische Basen wie beispielsweise Natriumbicarbonat, Natriumcarbonat oder die entsprechenden Kaliumsalze und deren Hydrate. Die im Reaktionsverlauf anfallenden anorganischen oder organischen Salze werden in bekannter Weise abgetrennt, z.B. unter Verwendung von Ionentauscher-Säuren und -Basen oder durch Filtration über bekannte Adsorbentien, wie z.B. Diaion oder Amberlite® XAD-2 und 4.

Die gegebenenfalls anschließende N-Alkylierung der aromatischen Acylaminogruppen erfolgt ebenfalls nach dem Fachmann bekannten Methoden, z.B. in polaren Lösungsmitteln wie Alkanolen oder Alkandiolen, wie Methanol, Ethanol oder Propandiol oder in Polyethern wie Ethylenglykoldiethylether, Diethylenglykoldimethylether u.a. oder deren Gemische in Gegenwart starker Basen, wie der Alkoholate von Natrium, Kalium oder deren Hydride.

Als Alkylierungsmittel dienen im Falle R³ in der Bedeutung niederes Alkyl oder Hydroxyalkyl die Alkyl- oder Hydroxyalkylhalogenide oder -sulfate bzw. deren Äquivalente. Beispielsweise Methyliodid, Methylbromid oder Dimethylsulfat für Verbindungen der Formel I mit R³ = Methyl; Ethylbromid, Ethyliodid oder Diethylsulfat für Verbindungen der Formel I mit R³ = Ethyl; Chlorethanol oder Bromethanol für Verbindungen der Formel I mit R³ = Hydroxyethyl; Chlorpropandiol oder Brompropandiol für Verbindungen der Formel I mit R³ = Dihydroxypropyl.

Die Spaltung vorhandener Schutzgruppen erfolgt unter den jeweils erforderlichen Bedingungen. Ketale werden zum Beispiel durch Mineralsäuren in einphasigen organisch-wäßrigen Lösungen bei Temperaturen zwischen 0°C und der Siedetemperatur der Reaktionsmischung gespalten. Besonders gut läßt sich ein Ketal in einer Tetrahydrofuran-Wasser-Mischung (1:1) durch Zugabe von halbkonzentrierter Salzsäure spalten. Der Temperaturbereich zwischen 10-40°C ist besonders geeignet.

Benzylether werden ebenfalls nach literaturbekanntern Methoden gespalten, zum Beispiel mit Natrium in Alkoholen oder flüssigem Ammoniak (Advances in Carbohydrat, Chem. 12, 149 [1957], J. Org. Chem 29, 3725 [1964].

Die Verseifung der Acyloxygruppen können in an sich bekannter Art und Weise erfolgen. Beispielsweise mit Basen in wäßrig-alkoholischer Lösung, so mit Natriumhydroxid in wäßrig-methanolischer Lösung.

Die verfahrensgemäß eingesetzten Ausgangsprodukte der allgemeinen Formel II können nach an sich bekannten Verfahren hergestellt werden, beispielsweise aus 5-Amino-2,4,6-triiod-isophthalsäuredichlorid (Deutsche Offenlegungsschrift 2031724).

### Herstellung der Ausgangsverbindungen

### Acetoxymalonsäure-bis[3,5-bis(chlorcarbonyl)-2,4,6-trijod-anilid]

119,14 g (200 mmol) 5-Amino-2,4,6-triiod-isophthalsäuredichlorid (Deutsche Offenlegungsschrift 2031724) werden in 1,2 l Toluol bei 100°C gelöst und zu dieser Lösung 21,9 g (110 mmol) Acetoxymalonylchlorid, gelöst in 50 ml Toluol, getropft. Es bildet sich sogleich ein kristalliner Niederschlag. Nach beendeter Zugabe wird das Heizbad entfernt, auf Raumtemperatur gekühlt und das Kristallisat abgesaugt. Man erhält 100,5 g = 76,3 mmol = 76,3 % der Theorie Acetoxymalonsäure-bis[3,5-bis(chlorcarbonyl)-2,4,6-triiod-anilid].

### Acetoxymalonsäure-bis[3,5-bis[chlorcarbonyl)-2,4,6-triiod-N-methylanilid]

182,9 g (300 mmol) 5-Methylamino-2,4,6-triiod-isophthalsäuredichlorid werden in 900 ml Toluol gelöst, die Lösung auf 85°C erwärmt und bei dieser Temperatur 32,83 g (165 mmol) Acetoxymalonylchlorid, gelöst in 65 ml Toluol, zugetropft. Das Produkt kristallisiert sofort aus der heißen Lösung aus. Es wird auf Raumtemperatur gekühlt, der Niederschlag abgesaugt und bei 50 °C im Vakuum getrocknet. Die Ausbeute beträgt 166,7 g = 123,9 mmol = 82,6 % der Theorie Acetoxymalonsäure-bis[3,5-bis(chlorcarbonyl)-2,4,6-triiod-N-methylanilid].

### Methoxymalonsäure-bis[3,5-bis(chlorcarbonyl)-2,4,6-triiod-N-methyl-anilid]

182,92 g (300 mmol) 5-Methylamino-2,4,6-triiod-isophthalsäuredichlorid werden in 900 ml Toluol gelöst, die Lösung auf 85°C erwärmt und innerhalb von 30 Minuten 28,2 g (165 mmol) Methoxymalonylchlorid zugetropft. Das Reaktionsprodukt kristallisiert sofort aus. Nach ca. 2 Stunden wird auf Raumtemperatur gekühlt, das Produkt abgesaugt, mit Toluol gewaschen und bei 50°C im Vakuum getrocknet. Die Ausbeute beträgt 155,14 g = 117,75 mmol = 78,5 % der Theorie Methoxymalonsäure-bis[3,5-(chlorcarbonyl)-2,4,6-triiod-N-methyl-anilid]

### Methoxymalonsäure-bis[3,5-bis(chlorcarbonyl)-2,4,6-triiod-anilid]

119,14 g (220 mmol) 5-Amino-2,4,6-triiod-isophthalsäuredichlorid werden in 1,2 l Toluol bei 100°C gelöst und zu dieser Lösung 18,8 g (110 mol) Methoxyacetylchlorid innerhalb von 30 Minuten getropft. Das Reaktionsprodukt fällt aus der Reaktionslösung kristallin aus. 1 Stunde nach Zugabe des Säurechlorids wird auf Raumtemperatur gekühlt, das Kristallisat abgesaugt, mit Toluol gewaschen und bei 50°C im Vakuum getrocknet. Die Ausbeute beträgt 102,64 g = 79,6 mmol = 79,6% der Theorie Methoxymalonsäure-bis[3,5-bis(chlorcarbonyl)-2,4,6-triiod-anilid].

### 2,3-Diacetoxy-bernsteinsäure-bis[3,5-bis(chlorcarbonyl)-2,4,6-triiod-N-methyl-anilid]

61 g (100 mmol) 5-Methylamino-2,4,6-triiod-isophthalsäuredichlorid werden in 600 ml Toluol gelöst, die Lösung auf 80°C erwärmt und 13,55 g (50 mmol) 2,3-Diacetoxy-bernsteinsäuredichlorid (D. Seebach et al. Ber. 1980, 1691), gelöst in 30 ml Toluol, zugetropft. Das Produkt kristallisiert schon bei erhöhter Temperatur aus. Nach 1 Stunde wird auf Raumtemperatur gekühlt, das Kristallisat abgesaugt, mit Toluol gewaschen und im Vakuum getrocknet. Die Ausbeute beträgt 51,5 g = 36,15 mmol = 72,3 % der Theorie 2,3-Diacetoxy-bernsteinsäure-bis[3,5-bis(chlorcarbonyl)-2,4,6-trijod-N-methyl-anilid].

### Benzyloxy-malonsäure-bis[3,5-bis(chlorcarbonyl)-2,4,6-triiod-anilid

70,4 g (130 mmol) 5-Amino-2,4,6-triiod-isophthalsäuredichlorid werden in 700 ml Toluol bei 100°C gelöst und zu dieser Lösung 16,06 g (65 mmol) Benzyloxymalonsäuredichlorid (hergestellt analog Hammond et al. Soc. 1957, 1062) zugefügt. Nach wenigen Minuten fällt das Bisanilid als kristalliner Niederschlag aus. Nach 30 Minuten wird die Heizung entfernt, auf Raumtemperatur gekühlt, der Niederschlag abgesaugt, mit Toluol gewaschen und bei 50°C im Vakuum getrocknet. Man erhält 60,18 g (44,07 mmol) = 67,8% der Theorie Kristallisat. Fp.: >350°C

### Benzyloxy-malonsäure-bis[3,5-bis(chlorcarbonyl)-2,4,6-triiod--N-methyl-anilid

45,75 g (75 mmol) 5-Methylamino-2,4,6-triiod-isophthalsäuredichlorid werden in 450 ml Toluol gelöst, die Lösung auf 80°C erwärmt und 9,4 g (38 mmol) Benzyloxymalonsäuredichlorid hinzugefügt. Das Bisanilid fällt nach wenigen Minuten kristallin aus. Nach einer Stunde wird auf Raumtemperatur gekühlt, der Niederschlag abgesaugt, mit Toluol gewaschen und bei 50°C im Vakuum getrocknet. Man erhält 38,4 g (27,56 mmol) = 73,5% der Theorie Kristallisat. Fp.: >350°C

### (2R,3R)-Di-O-benzoyl-weinsäure-bis[3,5-bis(chlorcarbonyl)-2,4,6-triiod-N-methyl anilid

30,4 g (50 mmol) 5-Methylamino-2,4,6-triiod-isophthalsäuredichlorid werden in 300 ml Toluol gelöst, die Lösung auf 80°C erwärmt und 21,93 g (25 mmol)(2R,3R)-Di-O-benzoyl-weinsäuredichlorid (hergestellt analog D. Seebach et al. Ber. 1980, 1691) hinzugefügt. Das Produkt beginnt nach wenigen Minuten kristllin auszufallen. Nach zwei Stunden wird auf Raumtemperatur gekühlt, der Niederschlag abgesaugt, mit Toluol gewaschen und bei 50°C im Vakuum getrocknet. Man erhält 56,74 g (36,8 mmol) = 73,6% der Theorie Kristallisat. Fp.: >350°C

### BEISPIEL 1

### Hydroxymalonsäure-bis[3,5-bis(2,3-dihydroxy-propylcarbamoyl)-2,4,6-triiod-anilid

263,5 g (200 mmol) Acetoxymalonsäure-bis[3,5-bis(chlorcarbonyl)-2,4,6-trijod-anilid] werden in 1,32 l Tetrahydrofuran gelöst und Lösung bei Raumtemperatur mit 164 g (1,8 mol) 1-Amino-propandiol-2,3, gelöst in 100 ml Tetrahydrofuran, versetzt. Das Produkt fällt zusammen mit dem Hydrochlorid des Amins aus. Nach fünfstündiger Reaktionszeit wird das Reaktionsgemisch unter vermindertem Druck eingedampft, der Rückstand in ca. 1 Liter Wasser gelöst, bei 50°C mit konzentrierter Natronlauge auf pH 12 gestellt, ca. 30 Minuten bei dieser Temperatur gerührt, dann mit konzentrierter Salzsäure neutralisiert und an Ionenaustauschern entsalzt. Das wäßrige Eluat wird unter vermindertem Druck zur Trockne eingedampft. Die Ausbeute beträgt 257,9 g = 172,6 mmol = 86,3 % der Theorie Hydroxymalonsäure-bis[3,5-bis(2,3-dihydroxy-propylcarbamoyl)-2,4,6-triiod-anilid].

| | | | | | | |
|---|---|---|---|---|---|---|
| Analyse: | Ber.: | C 24,92 | H 2,42 | J 50,96 | N 5,62 | O 16,06 |
| | Gef.: | C 25,13 | H 2,61 | J 50,67 | N 5,43 | |

### BEISPIEL 2

### Hydroxymalonsäure-bis[3,5-bis(2,3-dihydroxy-N-methyl-propylcarbamoyl)-2,4,6-triiod-anilid]

263,5 g (200 ml) Acetoxymalonsäure-bis[3,5-bis(chlorcarbonyl)-2,4,6-triiod-anilid] werden in 1,32 l Tetrahydrofuran gelöst. Zu dieser Lösung wird bei Raumtemperatur 189,3 g (1,8 mol) 1-Methylamino-propandiol-2,3, gelöst in 100 ml Tetrahydrofuran, getropft. Das Produkt fällt zusammen mit dem Hydrochlorid des Amins aus. Nach fünfstündiger Reaktionszeit wird das Reaktionsgemisch unter vermindertem Druck eingedampft, der Rückstand in ca. 1 Liter Wasser gelöst, bei 50°C mit konzentrierter Natronlauge auf pH 12 gestellt, ca. 30 Minuten bei dieser Temperatur gerührt, dann mit konzentrierter Salzsäure neutralisiert und an Ionenaustauschern entsalzt. Das wäßrige Eluat wird unter vermindertem Druck zur Trockne eingedampft. Die Ausbeute beträgt 269,73 g = 174 mmol = 87 % der Theorie Hydroxymalonsäure-bis[3,5-bis(2,3-dihydroxy-N-methyl-propylcarbamoyl)-2,4,6-triiod-anilid].

| | | | | | | |
|---|---|---|---|---|---|---|
| Analyse: | Ber.: | C 27,11 | H 2,86 | J 49,11 | N 5,42 | O 15,48 |
| | Gef.: | C 27,32 | H 2,67 | J 48,93 | N 5,40 | |

### BEISPIEL 3

### Hydroxymalonsäure-bis[3,5-bis[(1RS,2SR)-2,3-dihydroxy-1-hydroxymethylpropyl-carbamoyl]-2,4,6-triiod-anilid]

263,5 g (200 mmol) Acetoxymalonsäure-bis[3,5-bis(chlorcarbonyl)-2,4,6-triiod-anilid] werden in 1,32 l Tetrahydrofuran gelöst und Lösung bei Raumtemperatur 239,74 g (1,8 mol) 6-Amino-2,2-dimethyl-1,3-dioxepin-5-ol, gelöst in 200 ml Tetrahydrofuran, getropft. Nach einer fünfstündigen Reaktionszeit wird das Hydrochlorid abgesaugt, das Filtrat unter vermindertem Druck eingedampft, der Rückstand in ca. 1 Liter Wasser suspendiert, bei Raumtemperatur mit konzentrierter Salzsäure auf pH 1 angesäuert und 5 Stunden gerührt, dann mit konzentrierter Natronlauge auf pH 12 gestellt und 1 Stunde bei 50°C gerührt, schließlich mit konzentrierter Salzsäure neutralisiert und an Ionenaustauschern entsalzt. Das wäßrige Eluat wird unter vermindertem Druck zur Trockne eingedampft. Die Ausbeute beträgt 257 g = 159,2 mmol =79,6% der Theorie Hydroxymalonsäure-bis[3,5-bis[(1RS,2RS)-2,3-dihydroxy-1-hydroxymethylpropyl-carbamoyl]-2,4,6-trijod-anilid].

| | | | | | | |
|---|---|---|---|---|---|---|
| Analyse: | Ber.: | C 26,02 | H 2,80 | J 47,14 | N 5,20 | O 18,82 |
| | Gef.: | C 26,23 | H 2,88 | J 46,95 | N 5,13 | |

### BEISPIEL 4

### Hydroxymalonsäure-bis[3,5-bis(2,3-dihydroxy-propylcarbamoyl)-2,4,6-triiod-N-methyl-anilid]

269,1 g (200 mol) Acetoxymalonsäure-bis[3,5-bis(chlorcarbonyl)-2,4,6-triiod-N-methyl-anilid] werden in 1,32 l Tetrahydrofuran gelöst und zu dieser Lösung bei Raumtemperatur 164 g (1,6 mol) 1-Amino-propandiol-2,3, gelöst in 100 ml Tetrahydrofuran, getropft. Nach einer fünfstündigen Reaktionszeit wird das Reaktionsgemisch unter vermindertem Druck eingedampft, der Rückstand in ca. 1 Liter Wasser gelöst, bei 50°C mit konzentrierter Natronlauge auf pH 12 gestellt, ca. 45 Minuten bei dieser Temperatur gerührt, dann mit konzentrierter Salzsäure neutralisiert und an Ionenaustauschern entsalzt. Das wäßrige Eluat wird unter vermindertem Druck zur Trockne eingedampft. Die Ausbeute beträgt 258,8 g = 170 mmol = 85% der Theorie Hydroxymalonsäure-bis[3,5-bis(2,3-dihydroxy-propylcarbamoyl)-2,4,6-triiod-N-methyl-anilid].

| | | | | | | |
|---|---|---|---|---|---|---|
| Analyse: | Ber.: | C 26,04 | H 2,64 | J 50,02 | N 5,52 | O 15,76 |
| | Gef.: | C 25,93 | H 2,71 | J 49,87 | N 5,33 | |

### BEISPIEL 5

### Hydroxymalonsäure-bis[3,5-bis(2,3-dihydroxy-N-methyl-propylcarbamoyl)-2,4,6-triiod-N-methyl-anilid]

269,1 g (200 mmol) Acetoxymalonsäure-bis[3,5-bis(chlorcarbonyl)-2,4,6-triiod-N-methyl-anilid] werden in 1,35 l Tetrahydrofuran gelöst und zu dieser Lösung bei Raumtemperatur 189,3 g (1,6 mol) 1-Methylamino-propandiol-2,3, gelöst in 100 ml Tetrahydrofuran, zugetropft. Nach einer fünfstündigen Reaktionszeit wird das Reaktionsgemisch unter vermindertem Druck eingedampft, der Rückstand in ca. 1 Liter Wasser gelöst, bei 50°C mit konzentrierter Natronlauge auf pH 12 gestellt, ca. 50 Minuten bei dieser Temperatur gerührt, dann mit konzentrierter Salzsäure neutralisiert und an Ionenaustauschern entsalzt. Das wäßrige Eluat wird unter vermindertem Druck zur Trockne eingedampft. Die Ausbeute beträgt 162.6 g = 166.4 mmol = 83,2 % der Theorie Hydroxymalonsäure-bis[3,5-bis(2,3-dihydroxy-N-methyl-propylcarbamoyl)-2,4,6-triiod-N-methyl-anilid].

| | | | | | |
|---|---|---|---|---|---|
| Analyse: | Ber.: | C 28,15 | H 3,06 | J 48,24 | N 5,32 = 15,2 |
| | Gef.: | C 28,32 | H 3,17 | J 48,08 | N 5,2 |

### BEISPIEL 6

### Hydroxymalonsäure-bis[3,5-bis[(1RS,2SR)-2,3-dihydroxy-1-hydroxymethylpropyl-carbamoyl]-2,4,6-triiod-N-methyl-anilid]

2,69,1 g (200 mmol) Acetoxymalonsäure-bis[3,5-bis(chlorcarbonyl)-2,4,6-trijod-N-methyl-anilid] werden in 1,35 l Tetrahydrofuran gelöst und zu dieser Lösung bei Raumtemperatur 239,74 g (1,8 mol) 6-Amino-2,2-dimethyl-1,3-dioxepin-5-ol, gelöst in 200 ml Tetrahydrofuran, zugetropft. Nach einer fünfstündigen Reaktionszeit wird das Hydrochlorid abgesaugt, das Filtrat unter vermindertem Druck eingedampft, der Rückstand in ca. 1 l Wasser suspendiert, bei Raumtemperatur mit konzentrierter Salzsäure auf pH 1 angesäuert und 5 Stunden gerührt, dann mit konzentrierter Natronlauge auf pH 12 gestellt und eine Stunde bei 50°C gerührt, anschließend mit konzentrierter Salzsäure neutralisiert und an Ionenaustauschern entsalzt. Das wäßrige Eluat wird unter vermindertem Druck zur Trockne eingedampft. Die Ausbeute beträgt 277,2 g = 168,8 mmol = 84,4% der Theorie Hydroxymalonsäure-bis[3,5-bis[1RS,2SR)-2,3-dihydroxy-1-hydroxymethylpropyl-carbamoyl]-2,4,6-triiod-N-methyl-anilid].

| | | | | | | |
|---|---|---|---|---|---|---|
| Analyse: | Ber.: | C 27,06 | H 2,94 | J 46,36 | N 5,11 | O 18,51 |
| | Gef.: | C 26,93 | H 3,06 | J 46,15 | N 5,03 | |

### BEISPIEL 7

### Methoxymalonsäure-bis[3,5-bis(2,3-dihydroxy-propylcarbamoyl)-2,4,6-trijod-N-methyl-anilid]

262,35 g (200 mmol) Methoxymalonsäure-bis[3,5-bis(chlorcarbonyl)-2,4,6-triiod-N-Methyl-anilid] werden in 1,32 l Dioxan gelöst und zu dieser Lösung bei Raumtemperatur 164 g (1,8 mol) 1-Amino-propandiol-2,3, gelöst in 100 ml Dioxan, getropft. Nach 3 Stunden Reaktionszeit wird das Reaktionsgemisch unter vermindertem Druck eingedampft, der Rückstand in 800 ml Wasser gelöst und diese Lösung an Ionenaustauschern entsalzt. Das wäßrige Eluat wird nach der Entsalzung unter vermindertem Druck zur Trockne eingedampft. Die Ausbeute beträgt 262,07 g = 170,6 mmol = 85,3% der Theorie Methoxymalonsäure-bis[3,5dihydroxy-propylcarbamoyl)2,4,6-triiod-N-methyl-anilid].

| | | | | | | |
|---|---|---|---|---|---|---|
| Analyse: | Ber.: | C 26,58 | H 2,75 | J 49,56 | N 5,47 | O 15,62 |
| | Gef.: | C 26,43 | H 2,91 | J 49,37 | N 5,28 | |

### BEISPIEL 8

### Methoxymalonsäure-bis[3,5-bis(2,3-dihydroxy-propylcarbamoyl)-2,4,6-triiod-anilid]

128,95 g (100 mmol) Methoxymalonsäure-bis[3,5-bis(chlorcarbonyl)-2,4,6-triiod-anilid] werden in 644 ml Tetrahydrofuran gelöst und zu dieser Lösung bei Raumtemperatur 87,5 g (960 mmol) Aminopropandiol-2,3 - gelöst in 50 ml Tetrahydrofuran - getropft. Nach 5 Stunden Reaktion wird unter vermindertem Druck weitgehend eingeengt, der Rückstand wird in Wasser gelöst und an Ionenaustauschern entsalzt. Das eingedampfte Eluat der Ionenaustauscher ergibt 129,9 g =86,3 mmol = 86,3 % der Theorie amorphes Methoxymalonsäure-bis[2,3bis(2,3-dihydroxy-propylcarbamoyl)-2,4,6-triiod-anilid].

| | | | | | | |
|---|---|---|---|---|---|---|
| Analyse: | Ber.: | C 25,48 | H 2,54 | J 50,48 | N 5,57 | O 15,91 |
| | Gef.: | C 25,63 | H 2,71 | J 50,26 | N 5,32 | |

### BEISPIEL 9

### Methoxymalonsäure-bis[3,5-bis(2,3-dihydroxy-propylcarbamoyl)-2,4,6-triiod-N-methyl-anilid]

4,6 g (200 mmol) Natrium werden in einem Gemisch aus 200 ml Methanol und 200 ml Propandiol-1,2 gelöst, 75,4 g (50 mmol) Methoxymalonsäure-bis[3,5-bis(2,3-dihydroxy-propylcarbamoyl)-2,4,6-triiod-anilid] hinzugefügt, 3 Stunden bei 50°C gerührt, das Methanol dann bei Normaldruck abdestilliert, der verbleibenden Lösung 21,3 g (150 mmol) Methyliodid hinzugefügt und 24 Stunden bei 50°C gerührt. Die Reaktionslösung wird auf Raumtemperatur gekühlt und in 2 l Methylenchlorid eingerührt. Das Produkt fällt dabei als pastöse Masse aus. Von dieser wird dekantiert, in 200 ml Wasser gelöst und an Ionenaustauschern entsaltzt. Man erhält 59,76 g (38,9 mmol) = 77,8 % der Theorie Methoxymalonsäure-bis[3,5-bis(2,3-dihydroxy-propylcarbamoyl)-2,4,6-triiod-Nmethyl-anilid] als amorphen Feststoff.

| | | | | | | |
|---|---|---|---|---|---|---|
| Analyse: | Ber.: | C 26,58 | H 2,75 | J 49,56 | N 5,47 | O 15,62 |
| | Gef.: | C 26,67 | H 2,83 | J 49,37 | N 5,38 | |

### BEISPIEL 10

### 2,3-Dihydroxy-bernsteinsäure-bis[3,5-bis(2-hydroxy-1-hydroxymethylethyl)-2,4,6-triiod-anilid]

42,41 g (30 mmol) 2,3-Diacetoxy-bernsteinsäure-bis[3,5-bis(chlorcarbonyl)-2,4,6-triiod-N-methyl-anilid] werden in 420 ml Aceton suspendiert, 34,34 g(120 mml) Sodadekahydrat und 13,67 g (150 mmol) 2-Amino-propandiol-1,3 hinzugefügt, eine Stunde bei Raumtemperatur und zwei Stunden bei Siedetemperatur gerührt. Es wird dann auf Raumtemperatur gekühlt, der Niederschlag abgesaugt, mit 200 ml Ethanol heiß extrahiert, filtriert, das Ethanol- und Acetonfiltrat vereinigt und eingedampft. Der Rückstand wird in Wasser gelöst, die Acetatgruppen bei 50°C mit Natronlauge bei pH 11 verseift, mit Salzsäure neutralisiert, an Ionenaustauschern entsalzt und das Eluat gefriergetrocknet. Die Ausbeute beträgt 38,93 g = 25,08 mmol = 83,6 % der Theorie 2,3-Dihydroxy-bernsteinsäure-bis[3,5-bis(2-hydroxy-1-hydroxymethylethyl)-2,4,6-triiod-N-methyl-anilid].

| | | | | | | |
|---|---|---|---|---|---|---|
| Analyse: | Ber.: | C 25,21 | H 2,51 | J 49,95 | N 5,51 | O 16,79 |
| | Gef.: | C 25,07 | H 2,73 | J 49,7 | N 5,32 | |

### BEISPIEL 11

### Hydroxymalonsäure-bis[3,5-bis(2-hydroxy-1-hydroxymethylethyl)-2,4,6-triiod-N-(2 -hydroxyethyl)-anilid]

a) Benzyloxy-malonsäure-bis[3,5-bis(2-hydroxy-1-hydroxymethylethyl)-2,4,6-triiod-anilid]
68,3 g (50 mmol) Benzyloxy-malonsäure-bis[3,5-bis(chlorcarbonyl)-2,4,6-triiod-anilid wird in 140 ml Dimethylformamid gelöst, 25,3 g (250 mmol) Triethylamin hinzugefügt und bei Raumtemperatur 22,78 g (250 mmol) 2-Amino-propandiol-1,3 zugetropft. Man rührt 2 Stunden bei Raumtemperatur nach, saugt den Niederschlag des Triethylammoniumchlorids ab und tropft das Filtrat in 2 l Dichlormethan ein. Der feste amorphe Niederschlag wird abgesaugt, mit Dichlormethan gewaschen und in 300 ml Wasser bei Raumtemperatur suspendiert. Man rührt 24 Stunden bei Raumtemperatur, saugt den Feststoff ab, wäscht mit wenig Wasser nach und trocknet 60 Stunden bei 50°C im Vakuum. Man erhält 68,36 g (43,15 mmol) = 86,3% der Theorie des teils amorphen, teils kristallinen Feststoffs.

| | | | | | | |
|---|---|---|---|---|---|---|
| Analyse: | Ber.: | C 28,81 | H 2,67 | J 48,06 | N 5,30 | O 15,14 |
| | Gef.: | C 28,55 | H 2,48 | J 47,85 | N 5,53 | |

b) Hydroxy-malonsäure-bis[3,5-bis(2-hydroxy-1-hydroxymethylethyl)-2,4,6-triiod-N-(2-hydroxyethyl)-anilid]
4,83 g (210 mmol) Natrium werden in einem Gemisch von 200 ml Methanol und 200 ml Propandiol-1,2 gelöst, 79,2 g (50 mmol) des Produktes aus Beispiel 11a hinzugefügt, die Lösung 3 Stunden bei 50°C gerührt und das Methanol dann bei Normaldruck abdestilliert. Die Reaktionslosung wird darauf mit 16,43 g (200 mmol) Chlorethanol versetzt und 24 Stunden bei 50°C gerührt. Es wird auf Raumtemperatur gekühlt und in 3 l Aceton eingetropft. Der amorphe Niederschlag wird abgesaugt, mit Aceton gewaschen und 24 Stunden im Vakuum bei 50°C getrocknet. Das amorphe Benzyloxy-Zwischenprodukt wird in 1 l Ethanol in der Wärme gelöst und bei Raumtemperatur portionsweise 4,6 g (200 mmol) Natrium zugefügt. Die Lösung wird anschließend 12 Stunden bei Raumtemperatur gerührt und dann weitgehend eingedampft, der Rückstand in 150 ml Wasser gelöst, filtriert und am Ionenaustauscher entsalzt. Man erhält 45,1 g (28,5 mmol) = 57% der Theorie der Titelverbindung als amorphen Feststoff.

### BEISPIEL 12

### Hydroxy-malonsäure-bis[3,5-bis(2-hydroxy-1-hydroxymethylethyl)-2,4,6-triiod-N-methyl-anilid]

81,94 g (60 mmol) Benzyloxy-malonsäure-bis[3,5-bis(chlorcarbonyl)-2,4,6-triiod-N-methyl-anilid] werden in 160 ml Dimethylformamid gelöst, 30,4 g (300 mmol) Triethylamin zugetropft und bei Raumtemperatur 27,3 g (300 mmol) 2-Aminopropandiol-1,3 eingetropft. Mana rührt 2 Stunden bei Raumtemperatur, saugt den Niederschlag des Triethylammoniumchlorids ab und fällt das Filtrat in 2 l Dichlormethan. Die amorphe Fällung wird abgesaugt, mit Dichlormethan gewaschen und 24 Stunden bei 50°C im Vakuum getrocknet. Der Feststoff wird in 1 l Ethanol in der Wärme gelöst, die Lösung filtriert und bei Raumtemperatur portionsweise mit insgesamt 6,9 g (300 mmol) Natrium versetzt und anschließend 12 Stunden bei Raumtemperatur gerührt. Man dampft dann weitestgehend ein, löst in 200 ml Wasser und entsalzt an Ionenaustauschern. Die entsprechenden Eluatfraktoren ergeben eingedampft 58,07 g (38,1 mmol) = 63,5% der Theorie der Verbindungen als Feststoff.

| | | | | | | |
|---|---|---|---|---|---|---|
| Analyse: | Ber.: | C 26,00 | H 2,77 | J 49,95 | N 5,51 | O 15,74 |
| | Gef.: | C 26,18 | H 2,93 | J 49,72 | N 5,39 | |

### BEISPIEL 13

### 2-3-Dihydroxy-bernsteinsäure-bis[3,5-bis(2,3-dihydroxy-propycarbamoyl)-2,4,6-triiod-N-methyl-anild]

70,92 g (46 mmol) (2R,3R)-Di-O-benzoyl-weinsäure-bis[3,5-bis(chlorcarbonyl)-2,4,6-triiod-N-methyl-anilid] werden in 140 ml Dimethylformamid gelöst, 23,27 g (230 mmol) Triethylamin hinzugefügt und bei Raumtemperatur 21 g (230 mmol) 1-Aminopropandiol-2,3 eingetropft. Man rührt anschließend 2 Stunden bei Raumtemperatur, saugt dann den Niederschlag des Triethylammoniumchlorids ab und fällt das Filtrat in 2 l Dichlormethan. Die Fällung wird abgesaugt, mit Dichlormethan gewaschen und in 300 ml Wasser suspendiert. Diese Suspension wird bei 50°C mit Natronlauge bei pH 10-11 gehalten bis der pH-Wert nicht mehr abfällt. Es entsteht eine klare Lösung. Dann wird an Ionenaustauschern entsalzt. Die entsprechenden Eluatfraktionen werden zusammengefaßt und zur Trockne eingedampft. Man erhält 55,9 g (36 mmol) = 78,3% der Theorie als amorphen Feststoff.

| | | | | | | |
|---|---|---|---|---|---|---|
| Analyse: | Ber.: | C 26,31 | H 2,72 | J 49,05 | N 5,41 | O 16,49 |
| | Gef.: | C 26,47 | H 2,88 | J 48,87 | N 5,32 | |

## Patentansprüche (Patentansprüche für folgende(n) Vertragsstaat(en): AT, BE, CH, DE, FR, GB, GR, IT, LI, LU, NL, SE)

1. Substituierte Dicarbonsäure-bis(3,5-dicarbamoyl-2,4,6-triiodanilide) der allgemeinen Formel I 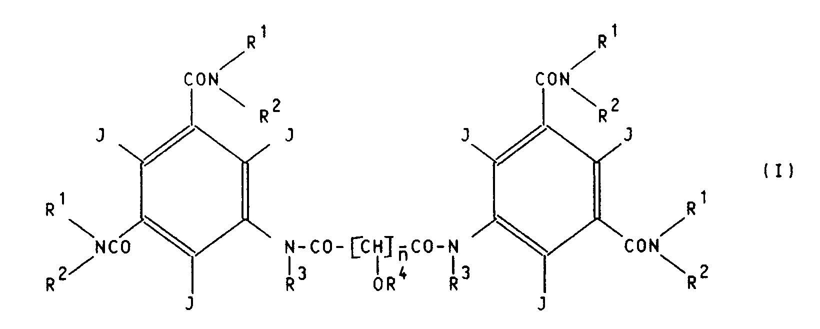 worin
R¹ ein Wasserstoffatom, einen C₁-C₄- Alkylrest oder R^{2,}
R² einen gerad- oder verzweigtkettigen C₂-C₈- Mono- oder Polyhydroxyalkylrest,
R³ ein Wasserstoffatom, einen C₁-C₄-Alkylrest oder R²
R⁴ ein Wasserstoffatom oder einen C₁-C₄-Alkylrest und
n = 1 oder 2
bedeuten.

2. Hydroxymalonsäure-bis[3,5-bis(2,3-dihydroxy-propylcarbamoyl)-2,4,6-triiod-anilid]
Hydroxymalonsäure-bis[3,5-bis(2,3-dihydroxy-N-methyl-propylcarbamoyl)-2,4,6-triiod-anilid]
Hydroxymalonsäure-bis[3,5-bis[1RS,2SR)-2,3-dihydroxy-1-hydroxy-methylpropylcarbamoyl]-2,4,6-triiod-anilid]
Hydroxymalonsäure-bis[3,5-bis(2,3-dihydroxy-propylcarbamoyl)-2,4,6-triiod-N-methyl-anilid]
Hydroxymalonsäure-bis[3,5-bis(2,3-dihydroxy-N-methyl-propylcarbamoyl)-2,4,6-triiod-N-methyl-anilid]
Hydroxymalonsäure-bis[3,5-bis[(1RS,2SR)-2,3-dihydroxy-1-hydroxy-methyl-propyl-carbamoyl]-2,4,6-triiod-N-methyl-anilid]
Methoxymalonsäure-bis[3,5-bis(2,3-dihydroxy-propylcarbamoyl)-2,4,6-triiod-N-methyl-anilid]
Methoxymalonsäure-bis[3,5-bis(2,3-dihydroxy-propylcarbamoyl)-2,4,6-triiod-anilid]
2,3-Dihydroxy-bersteinsäure-bis[3,5-bis(2-hydroxy-1-hydroxymethylethyl)-2,4,6-triiod-N-methyl-anilid]
Hydroxymalonsäure-bis[3,5-bis(2-hydroxy-1-hydroxymethylethyl)-2,4,6-triiod-N-(2-hydroxyethyl)-anilid]
Hydroxy-malonsäure-bis[3,5-bis(2-hydroxy-1-hydroxymethylethyl)-2,4,6-triiod-N-methyl-anilid]
2,3-Dihydroxy-bersteinsäure-bis[3,5-bis(2,3-dihydroxy-propycarbamoyl)-2,4,6 triiod-N-methyl-anild]

3. Verfahren zur Herstellung von Verbindungen der allgemeinen Formel I,
dadurch gekennzeichnet,
daß man in an sich bekannter Weise ein
substituiertes Dicarbonsäurederivat der allgemeinen Formel II 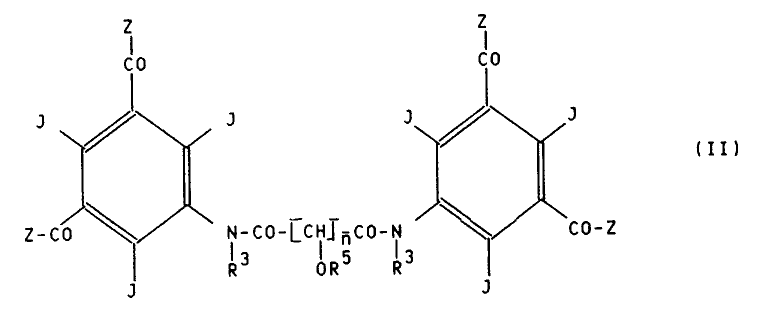 worin
R³ ein Wasserstoffatom oder eine C₁-C₄-Alkylgruppe,
R⁵ eine C₁-C₄-Alkylgruppe, Benzyl- oder eine C₁-C₆-Acylgruppe,
Z einen reaktiven Säure- oder Esterrest und
n = 1 oder 2
bedeuten,
mit einer Base der allgemeinen Formel III 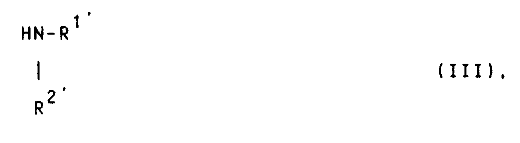 worin
R^{1'} ein Wasserstoffatom oder einen C₁-C₄-Alkylrest oder R^{2'} und
R^{2'} einen gerad- oder verzweigtkettigen C₂-C₈-Mono- oder Polyhydroxyalkylrest in freier oder geschützter Form
bedeuten,
umsetzt,
gegebenenfalls die aromatischen Acylaminogruppen zu C₁-C₄-N-Alkyl- oder N-Hydroxyalkyl-acylaminoverbindungen umsetzt und/oder gegebenenfalls die geschützten Hydroxylgruppen in Freiheit setzt.

4. Verwendung der Verbindungen gemäß Ansprüche 1 und 2 zur Herstellung von Röntgenkontrastmitteln.

## Patentansprüche (Patentansprüche für folgende(n) Vertragsstaat(en): ES)

1. Verfahren zur Herstellung von substituierten Dicarbonsäure-bis(3,5-dicarbamoyl-2,4,6-triiodaniliden) der allgemeinen Formel I 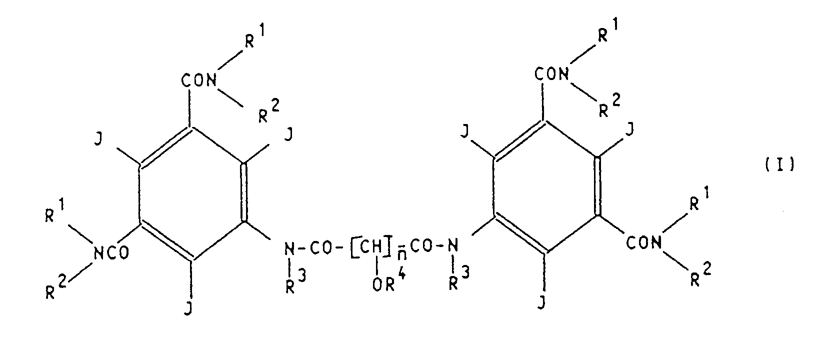 worin
R¹ ein Wasserstoffatom, einen C₁-C₄- Alkylrest oder R^{2,}
R² einen gerad- oder verzweigtkettigen C₂-C₈- Mono- oder Polyhydroxyalkylrest,
R³ ein Wasserstoffatom, einen C₁-C₄-Alkylrest oder R²
R⁴ ein Wasserstoffatom oder einen C₁-C₄-Alkylrest und
n = 1 oder 2
bedeuten, dadurch gekennzeichnet, daß man in an sich bekannter Weise ein substituiertes Dicarbonsäurederivat der allgemeinen Formel II 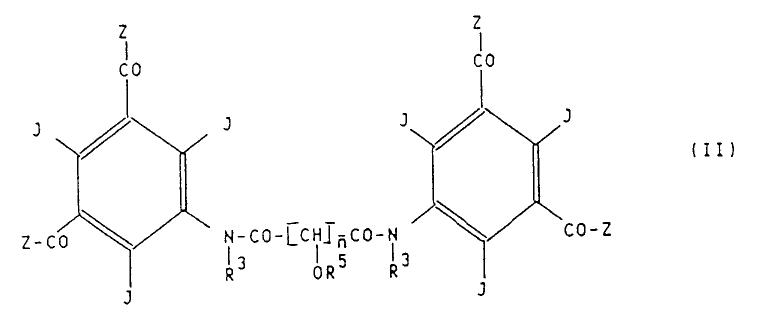 worin
R³ ein Wasserstoffatom oder eine C₁-C₄-Alkylgruppe,
R⁵ eine C₁-C₄-Alkylgruppe, Benzyl- oder eine C₁-C₆-Acylgruppe,
Z einen reaktiven Säure- oder Esterrest und
n = 1 oder 2
bedeuten,
mit einer Base der allgemeinen Formel III 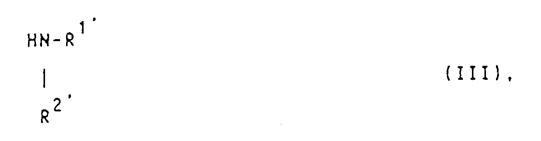 worin
R^{1'} ein Wasserstoffatom oder einen C₁-C₄-Alkylrest oder R^{2'} und
R^{2'} einen gerad- oder verzweigtkettigen C₂-C₈-Mono- oder Polyhydroxyalkylrest in freier oder geschützter Form
bedeuten,
umsetzt,
gegebenenfalls die aromatischen Acylaminogruppen zu C₁-C₄-N-Alkyl- oder N-Hydroxyalkyl-acylaminoverbindungen umsetzt und/oder gegebenenfalls die geschützten Hydroxylgruppen in Freiheit setzt.

2. Verfahren nach Anspruch 1, dadurch gekennzeichnet, daß als substituiertes Dicarbonsäure-bis(3,5-dicarbamoyl-2,4,6-triiodanilid
Hydroxymalonsäure-bis[3,5-bis(2,3-dihydroxy-propylcarbamoyl)-2,4,6-triiod-anilid]
Hydroxymalonsäure-bis[3,5-bis(2,3-dihydroxy-N-methyl-propylcarbamoyl)-2,4,6-triiod-anilid]
Hydroxymalonsäure-bis[3,5-bis[1RS,2SR)-2,3-dihydroxy-1-hydroxy-methylpropylcarbamoyl]-2,4,6-triiod-anilid]
Hydroxymalonsäure-bis[3,5-bis(2,3-dihydroxy-propylcarbamoyl)-2,4,6-triiod-N-methyl-anilid]
Hydroxymalonsäure-bis[3,5-bis(2,3-dihydroxy-N-methyl-propylcarbamoyl)-2,4,6-triiod-N-methyl-anilid]
Hydroxymalonsäure-bis[3,5-bis[(1RS,2SR)-2,3-dihydroxy-1-hydroxy-methyl-propyl-carbamoyl]-2,4,6-triiod-N-methyl-anilid]
Methoxymalonsäure-bis[3,5-bis(2,3-dihydroxy-propylcarbamoyl)-2,4,6-triiod-N-methyl-anilid]
Methoxymalonsäure-bis[3,5-bis(2,3-dihydroxy-propylcarbamoyl)-2,4,6-triiod-anilid]
2,3-Dihydroxy-bersteinsäure-bis[3,5-bis(2-hydroxy-1-hydroxymethylethyl)-2,4,6-triiod-N-methyl-anilid)
Hydroxymalonsäure-bis[3,5-bis(2-hydroxy-1-hydroxymethylethyl)-2,4,6-triiod-N-(2-hydroxyethyl)-anilid]
Hydroxy-malonsäure-bis[3,5-bis(2-hydroxy-1-hydroxymethylethyl)-2,4,6-triiod-N-methyl-anilid]
2,3-Dihydroxy-bersteinsäure-bis[3,5-bis(2,3-dihydroxy-propylcarbamoyl)-2,4,6-triiod-N-methyl-anilid]
hergestellt wird.

## Claims (Claims for the following Contracting State(s): AT, BE, CH, DE, FR, GB, GR, IT, LI, LU, NL, SE)

1. Substituted dicarboxylic acid bis(3,5-dicarbamoyl-2,4,6-triiodoanilides) of the general formula I 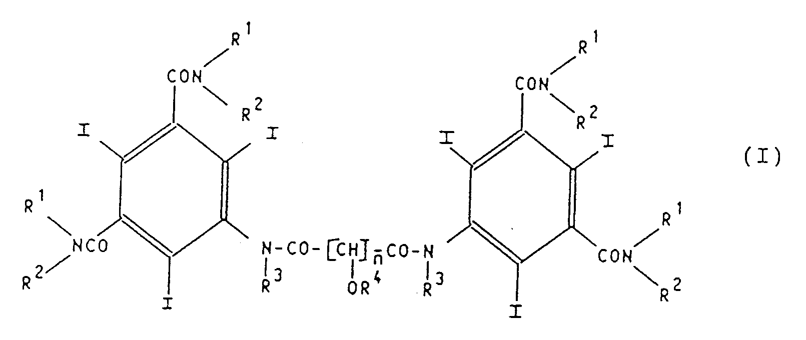 in which
R¹ represents a hydrogen atom, a C₁-C₄-alkyl radical or R²,
R² represents a straight-chain or branched-chain C₂-C₈-mono- or -poly-hydroxyalkyl radical,
R³ represents a hydrogen atom, a C₁-C₄-alkyl radical or R²,
R⁴ represents a hydrogen atom or a C₁-C₄-alkyl radical and
n = 1 or 2.

2. Hydroxymalonic acid bis[3,5-bis(2,3-dihydroxypropylcarbamoyl)-2,4,6-triiodoanilide]
hydroxymalonic acid bis[3,5-bis(2,3-dihydroxy-N-methylpropylcarbamoyl)-2,4,6-triiodoanilide]
hydroxymalonic acid bis[3,5-bis[(1RS,2SR)-2,3-dihydroxy-1-hydroxymethylpropylcarbamoyl]-2,4,6-triiodoanilide]
hydroxymalonic acid bis[3,5-bis(2,3-dihydroxypropylcarbamoyl)-2,4,6-triiodo-N-methylanilide]
hydroxymalonic acid bis[3,5-bis(2,3-dihydroxy-N-methylpropylcarbamoyl)-2,4,6-triiodo-N-methylanilide]
hydroxymalonic acid bis[3,5-bis[(1RS,2SR)-2,3-dihydroxy-1-hydroxymethylpropylcarbamoyl]-2,4,6-triiodo-N-methylanilide]
methoxymalonic acid bis[3,5-bis(2,3-dihydroxypropylcarbamoyl)-2,4,6-triiodo-N-methylanilide]
methoxymalonic acid bis[3,5-bis(2,3-dihydroxypropylcarbamoyl)-2,4,6-triiodoanilide]
2,3-dihydroxysuccinic acid bis[3,5-bis(2-hydroxy-1-hydroxymethylethyl)-2,4,6-triiodo-N-methylanilide]
hydroxymalonic acid bis[3,5-bis(2-hydroxy-1-hydroxymethylethyl)-2,4,6-triiodo-N-(2-hydroxyethyl)-anilide]
hydroxymalonic acid bis[3,5-bis(2-hydroxy-1-hydroxymethylethyl)-2,4,6-triiodo-N-methylanilide]
2,3-dihydroxysuccinic acid bis[3,5-bis(2,3-dihydroxypropylcarbamoyl)-2,4,6-triiodo-N-methylanilide].

3. Process for the preparation of compounds of the general formula I, characterised in that, in a manner known per se, a substituted dicarboxylic acid derivative of the general formula II 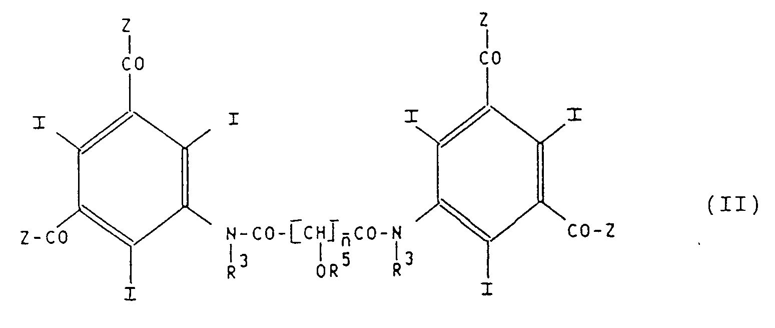 in which
R³ represents a hydrogen atom or a C₁-C₄-alkyl group,
R⁵ represents a C₁-C₄-alkyl group, a benzyl group or a C₁-C₆-acyl group,
Z represents a reactive acid radical or ester radical and
n = 1 or 2,
is reacted with a base of the general formula III 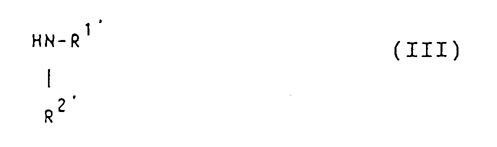 in which
R^{1'} represents a hydrogen atom or a C₁-C₄-alkyl radical or R^{2'} and
R^{2'} represents a straight-chain or branched-chain C₂-C₈-mono- or poly-hydroxyalkyl radical in free or protected form,
optionally the aromatic acylamino groups are reacted to form C₁-C₄-N-alkyl- or N-hydroxyalkyl-acylamino compounds and/or optionally the protected hydroxy groups are freed.

4. Use of the compounds according to claims 1 and 2 in the preparation of X-ray contrast media.

## Claims (Claims for the following Contracting State(s): ES)

1. Process for the preparation of substituted dicarboxylic acid bis(3,5-dicarbamoyl-2,4,6-triiodoanilides) of the general formula I 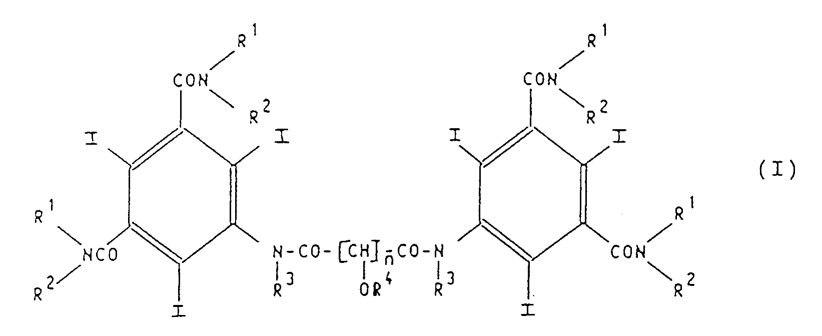 in which
R¹ represents a hydrogen atom, a C₁-C₄-alkyl radical or R²,
R² represents a straight-chain or branched-chain C₂-C₈-mono- or -poly-hydroxyalkyl radical,
R³ represents a hydrogen atom, a C₁-C₄-alkyl radical or R²,
R⁴ represents a hydrogen atom or a C₁-C₄-alkyl radical and
n = 1 or 2,
characterised in that, in a manner known per se, a substituted dicarboxylic acid derivative of the general formula II 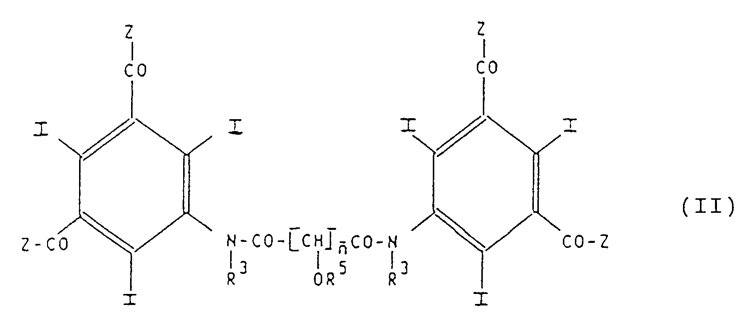 in which
R³ represents a hydrogen atom or a C₁-C₄-alkyl group,
R⁵ represents a C₁-C₄-alkyl group, a benzyl group or a C₁-C₆-acyl group,
Z represents a reactive acid radical or ester radical and
n = 1 or 2,
is reacted with a base of the general formula III 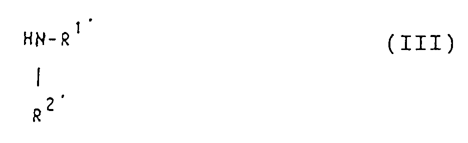 in which
R^{1'} represents a hydrogen atom or a C₁-C₄-alkyl radical or R^{2'} and
R^{2'} represents a straight-chain or branched-chain C₂-C₈-mono- or poly-hydroxyalkyl radical in free or protected form,
optionally the aromatic acylamino groups are reacted to form C₁-C₄-N-alkyl- or N-hydroxyalkyl-acylamino compounds and/or optionally the protected hydroxy groups are freed.

2. Process according to claim 1, characterised in that there is prepared as substituted dicarboxylic acid bis(3,5-dicarbamoyl-2,4,6-triiodoanilide)
hydroxymalonic acid bis[3,5-bis(2,3-dihydroxypropylcarbamoyl)-2,4,6-triiodoanilide]
hydroxymalonic acid bis[3,5-bis(2,3-dihydroxy-N-methylpropylcarbamoyl)-2,4,6-triiodoanilide]
hydroxymalonic acid bis[3,5-bis[(1RS,2SR)-2,3-dihydroxy-1-hydroxymethylpropylcarbamoyl]-2,4,6-triiodoanilide]
hydroxymalonic acid bis[3,5-bis(2,3-dihydroxypropylcarbamoyl)-2,4,6-triiodo-N-methylanilide]
hydroxymalonic acid bis[3,5-bis(2,3-dihydroxy-N-methylpropylcarbamoyl)-2,4,6-triiodo-N-methylanilide]
hydroxymalonic acid bis[3,5-bis[(1RS,2SR)-2,3-dihydroxy-1-hydroxymethylpropylcarbamoyl]-2,4,6-triiodo-N-methylanilide]
methoxymalonic acid bis[3,5-bis(2,3-dihydroxypropylcarbamoyl)-2,4,6-triiodo-N-methylanilide]
methoxymalonic acid bis[3,5-bis(2,3-dihydroxypropylcarbamoyl)-2,4,6-triiodoanilide]
2,3-dihydroxysuccinic acid bis[3,5-bis(2-hydroxy-1-hydroxymethylethyl)-2,4,6-triiodo-N-methylanilide]
hydroxymalonic acid bis[3,5-bis(2-hydroxy-1-hydroxymethylethyl)-2,4,6-triiodo-N-(2-hydroxyethyl)-anilide]
hydroxymalonic acid bis[3,5-bis(2-hydroxy-1-hydroxymethylethyl)-2,4,6-triiodo-N-methylanilide]
2,3-dihydroxysuccinic acid bis[3,5-bis(2,3-dihydroxypropylcarbamoyl)-2,4,6-triiodo-N-methylanilide].

## Revendications (Revendications pour l'(les) Etat(s) contractant(s) suivant(s): AT, BE, CH, DE, FR, GB, GR, IT, LI, LU, NL, SE)

1. Bis-(3,5-dicarbamoyl-2,4,6-triiodanilides) d'acide dicarboxylique de la formule générale I 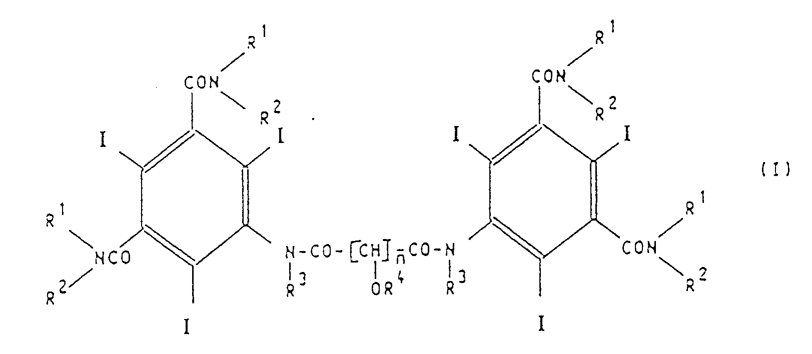 dans laquelle
R¹ représente un atome d'hydrogène, un radical alkyle en C₁-C₄ ou R²,
R² représente un radical monohydroxyalkyle ou polyhydroxyalkyle en C₂-C₈, à chaîne linéaire ou ramifiée,
R³ représente un atome d'hydrogène, un radical alkyle en C₁-C₄ ou R²,
R⁴ représente un atome d'hydrogène ou un radical alkyle en C₁-C₄, et
n = 1 ou 2.

2. Bis-[3,5-bis-(2,3-dihydroxy-propylcarbamoyl)-2,4,6-triiodo-anilide] d'acide hydroxymalonique,
bis-[3,5-bis-(2,3-dihydroxy-N-méthyl-propylcarbamoyl)-2,4,6-triiodo-anilide] d'acide hydroxymalonique,
bis-[3,5-bis-[(1RS,2SR)-2,3-dihydroxy-1-hydroxy-méthyl-propylcarbamoyl]-2,4,6-triiodo-anilide] d'acide hydroxymalonique,
bis-[3,5-bis-(2,3-dihydroxy-propylcarbamoyl)-2,4,6-triiodo-N-méthyl-anilide] d'acide hydroxymalonique,
bis-[3,5-bis-(2,3-dihydroxy-N-méthyl-propylcarbamoyl)-2,4,6-triiodo-N-méthyl-anilide] d'acide hydroxymalonique,
bis-[3,5-bis-[(1RS,2SR)-2,3-dihydroxy-1-hydroxy-méthyl-propyl-carbamoyl]-2,4,6-triiodo-N-méthyl-anilide] d'acide hydroxymalonique,
bis-[3,5-bis-(2,3-dihydroxy-propylcarbamoyl)-2,4,6-triiodo-N-méthyl-anilide] d'acide méthoxymalonique,
bis-[3,5-bis-(2,3-dihydroxy-propylcarbamoyl)-2,4,6-triiodo-anilide] d'acide méthoxymalonique,
bis-[3,5-bis-(2-hydroxy-1-hydroxyméthyléthyl)-2,4,6-triiodo-N-méthyl-anilide] d'acide 2,3-dihydroxy-succinique,
bis-[3,5-bis-(2-hydroxy-1-hydroxyméthyléthyl)-2,4,6-triiodo-N-(2-hydroxyéthyl)-anilide] d'acide hydroxymalonique,
bis-[3,5-bis-(2-hydroxy-1-hydroxyméthyléthyl)-2,4,6-triiodo-N-méthyl-anilide] d'acide hydroxymalonique,
bis-[3,5-bis-(2,3-dihydroxy-propylcarbamoyl)-2,4,6-triiodo-N-méthyl-anilide] d'acide 2,3-dihydro-succinique.

3. Procédé de préparation de composés de la formule générale I, caractérisé en ce que, d'une manière connue en soi, on fait réagir un dérivé d'acide dicarboxylique substitué de la formule générale II 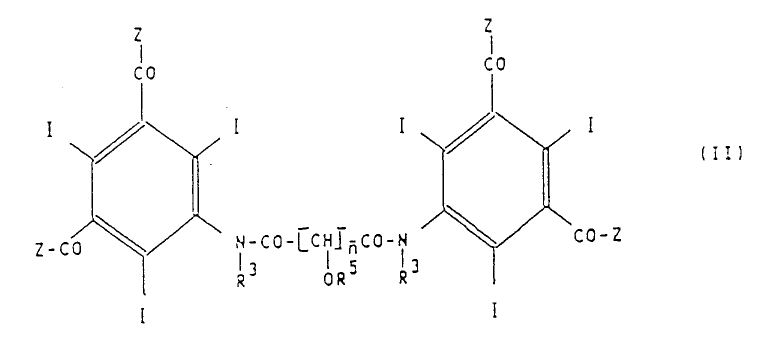 dans laquelle
R³ représente un atome d'hydrogène ou un groupe alkyle en C₁-C₄,
R⁵ représente un groupe alkyle en C₁-C₄, un groupe benzyle ou un groupe acyle en C₁-C₆,
Z représente un radical ester ou acide réactif, et
n = 1 ou 2,
avec une base de la formule générale III 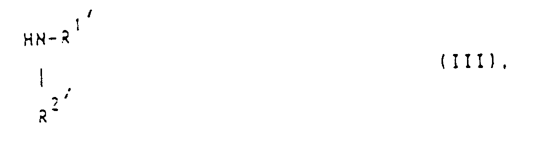 dans laquelle
R^{1'} représente un atome d'hydrogène ou un radical alkyle en C₁-C₄ ou R^{2'}, et
R^{2'} représente un radical monohydroxyalkyle ou polyhydroxyalkyle en C₂-C₈, à chaîne linéaire ou ramifiée,
sous forme libre ou protégée,
et on fait éventuellement réagir les groupes acylamino aromatiques pour former des composés N-alkyl-acylamino ou N-hydroxyalkyl-acylamino où le groupe alkyle est en C₁-C₄, et/ou on libère éventuellement les groupes hydroxy protégés.

4. Utilisation des composés suivant l'une des revendications 1 et 2, pour la préparation de produits de contraste aux rayons X.

## Revendications (Revendications pour l'(les) Etat(s) contractant(s) suivant(s): ES)

1. Procédé de préparation de bis-(3,5-dicarbamoyl-2,4,6-triiodanilides) d'acide dicarboxylique substitués de la formule générale I 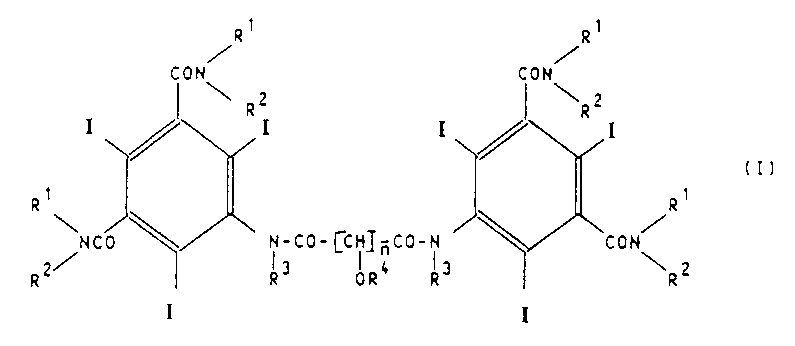 dans laquelle
R¹ représente un atome d'hydrogène, un radical alkyle en C₁-C₄ ou R²,
R² représente un radical monohydroxyalkyle ou polyhydroxyalkyle en C₂-C₈, à chaîne linéaire ou ramifiée,
R³ représente un atome d'hydrogène, un radical alkyle en C₁-C₄ ou R²,
R⁴ représente un atome d'hydrogène ou un radical alkyle en C₁-C₄, et
n = 1 ou 2,
caractérisé en ce que, d'une manière connue en soi, on fait réagir un dérivé d'acide dicarboxylique substitué de la formule générale II 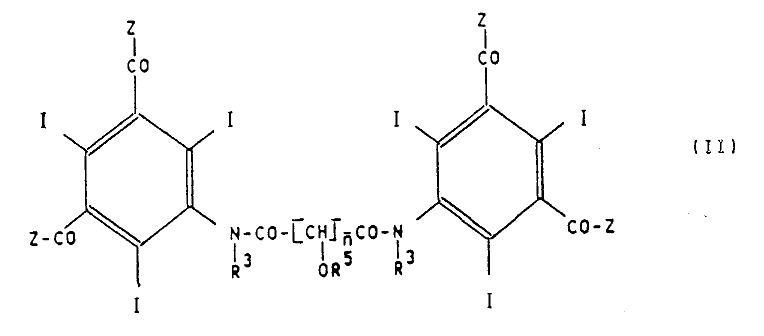 dans laquelle
R³ représente un atome d'hydrogène ou un groupe alkyle en C₁-C₄,
R⁵ représente un groupe alkyle en C₁-C₄, un groupe benzyle ou un groupe acyle en C₁-C₆,
Z représente un radical ester ou acide réactif, et
n = 1 ou 2,
avec une base de la formule générale III 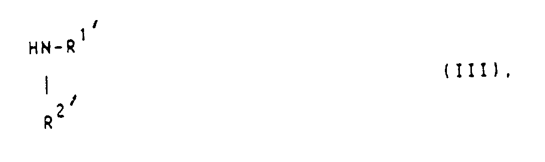 dans laquelle
R^{1'} représente un atome d'hydrogène ou un radical alkyle en C₁-C₄ ou R^{2'}, et
R^{2'} représente un radical monohydroxyalkyle ou polyhydroxyalkyle en C₂-C₈, à chaîne linéaire ou ramifiée, sous forme libre ou protégée,
et on fait éventuellement réagir les groupes acylamino aromatiques pour former des composés N-alkyl-acylamino ou N-hydroxyalkyl-acylamino où le groupe alkyle est en C₁-C₄, et/ou on libère éventuellement les groupes hydroxy protégés.

2. Procédé suivant la revendication 1, caractérisé en ce que, comme bis-(3,5-dicarbamoyl-2,4,6-triiodoanilide) d'acide dicarboxylique substitué, on prépare
du bis-[3,5-bis-(2,3-dihydroxy-propylcarbamoyl)-2,4,6-triiodo-anilide] d'acide hydroxymalonique,
du bis-[3,5-bis-(2,3-dihydroxy-N-méthyl-propylcarbamoyl)-2,4,6-triiodo-anilide] d'acide hydroxymalonique,
du bis-[3,5-bis-[(1RS,2SR)-3,4-dihydroxy-1-hydroxyméthylpropylcarbamoyl]-2,4,6-triiodo-anilide] d'acide hydroxymalonique,
du bis-[3,5-bis-(2,3-dihydroxy-propylcarbamoyl)-2,4,6-triiodo-N-méthyl-anilide] d'acide hydroxymalonique,
du bis-[3,5-bis-(2,3-dihydroxy-N-méthyl-propylcarbamoyl)-2,4,6-triiodo-N-méthyl-anilide] d'acide hydroxymalonique,
du bis-[3,5-bis[(1RS,2SR)-2,3-dihydroxy-1-hydroxyméthyl-propyl-carbamoyl]-2,4,6-triiodo-N-méthyl-anilide] d'acide hydroxymalonique,
du bis-[3,5-bis-(2,3-dihydroxy-propylcarbamoyl)-2,4,6-triiodo-N-méthyl-anilide] d'acide méthoxymalonique,
du bis-[3,5-bis-(2,3-dihydroxy-propylcarbamoyl)-2,4,6-triiodo-anilide] d'acide méthoxymalonique,
du bis-[3,5-bis-(2-hydroxy-1-hydroxyméthyléthyl)-2,4,6-triiodo-N-méthyl-anilide] d'acide 2,3-dihydroxy-succinique,
du bis-[3,5-bis-(2-hydroxy-1-hydroxyméthyléthyl)-2,4,6-triiodo-N-(2-hydroxyéthyl)-anilide] d'acide hydroxymalonique,
du bis-[3,5-bis-(2-hydroxy-1-hydroxyméthyléthyl)-2,4,6-triiodo-N-méthyl-anilide] d'acide hydroxymalonique,
du bis-[3,5-bis-(2,3-dihydroxy-propylcarbamoyl)-2,4,6-triiodo-N-méthyl-anilide] d'acide 2,3-dihydro-succinique.
